# EUROPÄISCHE PATENTSCHRIFT

(11) **EP 1 844 710 B1**
(45) Veröffentlichungstag und Bekanntmachung des Hinweises auf die Patenterteilung: **15.10.2008**
(21) Anmeldenummer: 06007800.3
(22) Anmeldetag: 13.04.2006
(51) Int. Cl.: A61B 5/15

(54) **Lanzettenvorrichtung zum Punktieren der Haut**
Lancet for skin puncture
Lancette pour perforer la peau

(43) Veröffentlichungstag der Anmeldung: 17.10.2007
(73) Patentinhaber: Haemedic AB, 135 48 Tyresö (SE)
(72) Erfinder: Steg, Henning, S-13550 Tyresö (SE)
(74) Vertreter: Niedmers, Ole

(56) Entgegenhaltungen:
- EP-A- 0 678 278
- EP-A- 1 247 489
- WO-A-20/05094680
- WO-A-20/05110227
- US-A- 4 653 513
- US-A- 5 540 709
- US-B1- 6 852 119

## Beschreibung

Die Erfindung betrifft eine Lanzettenvorrichtung zum Punktieren der Haut von Säugetieren, insbesondere des Menschen, umfassend ein mit einer Handhabe versehenes Steckelement sowie ein mit einer Handhabe versehenes Buchsenelement zur inneren, axial verschiebbaren Aufnahme des Steckelementes, wobei das Steckelement an seinem von der Handhabe weggerichteten freien Ende eine mit einem spitzen Ende versehene Lanzette zum gezielten Eintritt in die Haut aufweist, und wobei die Lanzette über das Steckelement durch eine in axialer Richtung auf das freie Buchsenende hin wirkende Kraft mit einem voreinstellbaren Impuls aus dem Buchsenende heraus in die Haut eintreten läßt, und daß Blockierungsmittel vorgesehen sind, die nach einmaligem Eintritt der Lanzette in die Haut die Möglichkeit eines nochmaligen Eintritts verhindern.

Eine Lanzettenvorrichtung dieser Art ist bekannt (EP-A-0 565 819). Dokument EP 1 247 489 offenbart eine Lanzettenvorrichtung gemäß dem Oberbegriff des Anspruchs 1. Lanzettenvorrichtung zum gezielten Punktieren der Haut, insbesondere des Menschen, sind in den unterschiedlichsten Ausführungsformen seit langem bekannt und werden mit mehr oder weniger gutem Erfolg seit langem im ambulanten Bereich, in medizinischen Einrichtungen, in Krankenhäusern, in Arztpraxen, bei Rote-Kreuz-Einrichtungen und auch bei Katastrophenschutzeinrichtungen und dergleichen eingesetzt, um beispielsweise geringe Mengen Blutes für Blutuntersuchungen zur Verfügung zu haben. Wesentliche Voraussetzungen bei diesen Lanzettenvorrichtungen ist es, daß diese sehr kostengünstig bereitstellbar sein müssen, da sie vielfach in großen Mengen von den voraufgeführten Institutionen verwendet werden und dort auch in großen Mengen bereitgehalten werden müssen und auch gezielt in den Ländern der Dritten Welt eingesetzt werden sollen.

Eine weitere Forderung an diese Lanzettenvorrichtung ist die, daß zumindest die Teile der Lanzettenvorrichtung, die unmittelbar die Haut des Menschen durchstoßen, also unmittelbar Kontakt mit dem unter der Außenhaut liegenden Gewebe und dem Blut des Menschen haben, fortwährend bis zum Einsatz der Lanzettenvorrichtung absolut steril gehalten werden müssen. In der Regel handelt es sich bei den Teilen der Lanzettenvorrichtung, die in das Gewebe in einer vorbestimmbaren Tiefe eindringen sollen, um Blutgefäße zu treffen und das Blut aus der Eintrittsöffnung der Wunde austreten zu lassen, um sogenannte Lanzetten, die aus einem im wesentlichen einen kreisförmigen Querschnitt aufweisenden Stahlkörper bestehen, der an seinem zu der zu punktierenden Haut weisenden Ende angespitzt ist.

Die obige gattungsgemäße Lanzettenvorrichtung weist Mittel auf, die verhindern, daß die Lanzettenvorrichtung nach erfolgtem bestimmungsgemäßen Gebrauch nicht noch einmal 1 verwendet werden kann.

Lanzettenvorrichtungen, die diese Verriegelungsmittel nicht aufweisen, können grundsätzlich erneut verwendet werden, was zu erheblichen diagnostischen Fehlern aber auch, was noch sehr viel gravierender ist, zu Infektionen des Benutzers der Lanzettenvorrichtung aufgrund von nicht beseitigten Keimen des Vorbenutzers führen kann.

Die Tendenz geht aus den vorgenannten Gründen dahin, nur noch solche Lanzettenvorrichtungen einzusetzen, die tatsächlich nur einmal verwendet werden können, d.h. eine nochmalige Verwendung, selbst wenn man das wollte, durch konstruktive Maßnahmen in der Lanzettenvorrichtung verhindern, und zwar immer dann, wenn eine nochmalige Verwendung der Lanzettenvorrichtung durch die Benutzerkreise nicht in jedem Falle ausgeschlossen werden kann. In vielen Ländern dürfen aufgrund gesetzlicher Vorschriften generell nur einmal benutzbare Lanzettenvorrichtungen eingesetzt werden.

Die bisher im Stand der Technik bekannten Lanzettenvorrichtungen, die durch Vorsehen konstruktiver Mittel nur eine einmalige Benutzung der Lanzettenvorrichtung erlauben, sind durch einen verhältnismäßig komplexen Aufbau gekennzeichnet. Die Funktionsfähigkeit dieser bekannten Lanzettenvorrichtungen, die nur einmal ausgelöst bzw. verwendet werden können, ist regelmäßig gewährleistet, sie haben jedoch einen erheblichen Nachteil, der darin liegt, daß ihre Gestehungskosten im Vergleich zu den mehrfach auslösbaren bzw. verwendbaren Lanzettenvorrichtungen erheblich höher liegen. Das führt dazu, daß diese nur einmal auslösenden Lanzettenvorrichtungen nicht in der Breite von den ins Auge gefaßten Benutzerkreisen eingesetzt werden, wie dieses aus gesundheitlichen und gesündheitspolitischen Gründen an sich erwünscht ist.

Es ist deshalb Aufgabe der vorliegenden Erfindung eine Lanzettenvorrichtung der Eingangs genannten Art zu schaffen, d.h. eine solche, die nur zum einmaligen Gebrauch befähigt ist und einen nochmaligen Gebrauch nicht ermöglicht, die sehr einfach im Aufbau ist und kostengünstiger als bisherige Lanzettenvorrichtungen dieser Art herstellbar und bereitstellbar ist, die darüber hinaus bis unmittelbar vor dem Einsatz eine absolute Sterilität der in die Haut des Menschen eindringenden Teile der Lanzettenvorrichtung sicherstellt, die fortwährend eine von den individuellen Eigenschaften der Bedienungsperson unabhängige, gleichmäßige Eintrittsfunktion der Lanzettenspitze in die Haut gewährleistet und die darüber hinaus nach ausgeführter Punktierung der Haut sicherstellt, daß die Lanzettenspitze abgeschirmt wird und eine unbeabsichtigte Verletzung und damit eine einhergehende Infektion durch an der Lanzettenspitze anhaftendes Blut verhindert.

Gelöst wird die Aufgabe gemäß der Erfindung gemäß Anspruch 1 dadurch, daß im Innenraum des Buchsenelementes ein axial in diesem verschiebbares Hülsenelement angeordnet ist, das einen im wesentlichen auf die Achse hin gerichteten Vorsprung aufweist, über den hinweg ein von der Achse weggerichteter erster Steckelementvorsprung im Zuge des Eintritts der Lanzette in die Haut derart gleitend verschiebbar ist, daß er axial hinter den Vorsprung greift und im Zuge des Wiedereintritts der Lanzette in das Buchsenende das Hülsenelement axial in Richtung der Handhabe verschiebt.

Der Vorteil der erfindungsgemäßen Lösung besteht im wesentlichen darin, daß durch die erfindungsgemäß vorgeschlagenen konstruktiven Maßnahmen keine Möglichkeit für den Benutzer einer derartigen Lanzettenvorrichtung besteht, diese nach einmaligem Gebrauch nochmals auszulösen bzw. nochmals benutzen zu können. Aufgrund der erfindungsgemäß konstruktiven Maßnahme ist es nicht möglich, die Lanzettenvorrichtung nach einmaligem Gebrauch bzw. einmaliger Auslösung überhaupt noch einmal auszulösen, da besagtes Hülsenelement nach Auslösung an der Handhabe des Steckelementes, das die eigentliche Lanzette aufnimmt, unverrückbar anliegt und somit die Handhabe des Steckelementes in axialer Richtung nicht mehr bewegbar ist.

Wie aufgabengemäß ebenfalls angestrebt wird, ist durch die erfindungsgemäßen Maßnahmen sichergestellt, daß nach Gebrauch der Lanzettenvorrichtung die Lanzettenspitze nach Wiedereintritt in das Buchsenende unverrückbar abgeschirmt ist, d.h. die Lanzettenspitze kann weder beabsichtigt noch unbeabsichtigt berührt werden, so daß eine Infektion Dritter durch an der Lanzettenspitze anhaftendes Blut nicht mehr möglich ist. Schließlich zeichnet sich die erfindungsgemäße Lösung, wie aufgabengemäß angestrebt, durch eine sehr einfache aber hocheffektive Konstruktion aus, da diese gegenüber den Lanzettenvorrichtungen, die bei ansonsten gleichem Aufbau wie die gattungsgemäßen Lanzettenvorrichtungen zur Mehrfachauslösung befähigten Lanzettenvorrichtungen nur ein Element, nämlich das Hülsenelement, mehr aufweist. Die Gestehungskosten der erfindungsgemäßen Lanzettenvorrichtung liegen somit nur geringfügig über den der Lanzettenvorrichtungen gleichen Aufbaus, die keine Mittel aufweisen, mit denen eine nochmalige Auslösung der Lanzettenvorrichtung nach erfolgtem Gebrauch verhindert wird.

Gemäß einer vorteilhaften Ausgestaltung der Lanzettenvorrichtung weist das Hülsenelement an seinem dem ersten Vorsprung im wesentlichen entgegengesetzten Ende einen Absatz auf, der nach Abschluß der axialen Verschiebung in Richtung der Handhabe hinter einen am Buchsenelement innen ausgebildeten Buchsenabsatz greift. Die Verriegelung der Lanzettenvorrichtung nach einmaligem Gebrauch erfolgt somit letztlich zwischen dem Hülsenelement und dem Buchsenelement, das in Einziehrichtung der Lanzette in das Buchsenelement soweit axial verschoben wird, bis der Absatz des Hülsenelementes hinter den Buchsenabsatz greift. Dabei kommt das Hülsenelement an der Handhabe des Steckelementes innen zuliegen, so daß die Handhabe des Steckelementes nicht mehr bewegbar ist.

Es gibt eine Reihe von Möglichkeiten konstruktiver Art sicherzustellen, daß das Hülsenelement nach Abschluß der axialen Verschiebung in Richtung der Handhabe hinter den am Buchsenelement innen ausgebildeten Buchsenabsatz greift, beispielsweise vorteilhafterweise dadurch, daß das Hülsenelement wenigstens im Bereich seines Absatzes radial von der Achse weg vorgespannt ist. Das kann beispielsweise dadurch bewirkt werden, daß das Hülsenelement während des Herstellungsvorganges in radialer Richtung derart bemessen wird, das es, seine elastische Verformbarkeit ausnutzend, in den durch das Buchsenelement gebildeten Innenraum im Zuge der Montage, geringfügig radial, d.h. in seinem Durchmesser, vermindert wird und dann in den Innenraum eingeführt wird. Die eigene Betätigung zur elastischen Rückverformung des Hülsenelementes wird dann ausgenutzt, so daß das Hülsenelement zunächst wenigstens teilweise an der Innenwandung des Buchsenelementes anliegt und sich nach besagter axialer Verschiebung radial nach außen vergrößert und somit hinter den Buchsenabsatz greift.

Bei einer noch anderen vorteilhaften Ausgestaltung der Lanzettenvorrichtung weist das Steckelement an seinem freien Ende ein die daraus herausstehende Lanzettenspitze steril umhüllendes Abdeckelement auf, was den Vorteil hat, daß das Abdeckelement erst unmittelbar vor dem Gebrauch der Lanzettenvorrichtung von der Lanzettenspitze entfernt zu werden braucht und durch dieses Abdeckelement die Lanzettenspitze eine faktisch unbegrenzte zeitlang steril gehalten werden kann.

Bei einer noch weiteren vorteilhaften Ausgestaltung der Lanzettenvorrichtung sind das Steckelement und das Abdeckelement als einstückiges Formteil ausgebildet mit der Folge, daß diese Ausgestaltung in einem Arbeitsgang und somit kostengünstig herstellbar ist.

Um das Abdeckelement für den Punktierungsvorgang mit der Lanzettenvorrichtung leicht und schnell vom Steckelement entfernen und so die Lanzettenspitze freilegen zu können, ist das Steckelement vorzugsweise im Bereich des Anschlusses an das Abdeckelement mit einer Sollbruchstelle versehen. Die Sollbruchstelle selbst gestattet ein schnelles Entfernen des Abdeckelements, vorzugsweise dann, wenn die Sollbruchstelle vorteilhafterweise durch eine radiale Verjüngung des Anschlußbereiches gebildet wird, so daß das Abdeckelement gegenüber dem Steckelement lediglich zu seiner Entfernung geringfügig um einen Winkel α gedreht zu werden braucht und so die Verbindung zwischen Steckelement und Abdeckelement, d.h. in der Verjüngung des Anschlußbereiches, bricht.

Um zu verhindern, daß die Lanzettenvorrichtung im unbenutzten Zustand unbeabsichtigt ausgelöst wird, weist gemäß einer anderen vorteilhaften Ausgestaltung das Abdeckelement an seinem in unausgelöstem Zustand der Lanzettenvorrichtung im Buchsenelement positionierten Ende im wesentlichen wenigstens zwei vom Abdeckelement im wesentlichen radial abstehende Verriegelungsstege auf, die im unausgelösten Zustand der Lanzettenvorrichtung bei Betätigung der Handhabe in axialer Richtung an ein im Bereich des freien Endes des Buchsenelementes ausgebildetes Widerlager stoßen. Somit ist es unmöglich, die Lanzettenvorrichtung unbeabsichtigt auszulösen, solange das Abdeckelement die Lanzettenspitze steril bedeckt bzw. einfaßt.

Vorzugsweise stehen bei Verdrehung des Abdeckelementes um die Achse um einen Winkel α vorbestimmbarer Größe die Verriegelungsstege einem widerlagerfreien Abschnitt im Bereich des freien Endes des Buchsenelementes gegenüber, wobei aufgrund der axialen Drehung des Abdeckelementes um den Winkel α, das Abdeckelement wird dabei beispielsweise für die Drehung mit Daumen und Zeigefinger eines Benutzers ergriffen und gedreht, die Verbindung zwischen Abdeckelement und dem Steckelement gelöst wird und das Abdeckelement aus dem Buchsenelement herausgezogen werden kann.

Vorteilhafterweise weist das Steckelement einen von der Achse weggerichteten zweiten, in Austrittsrichtung der Lanzette vor dem ersten Steckelementvorsprung ausgebildeten Steckelementvorsprung und axial zu diesem beabstandet auf, der den axialen Verschiebungsweg der Lanzette in das Buchsenelement entgegen der Austrittsrichtung der Lanzette in Anlage an einen Absatz des Buchsenelementes kommend, begrenzt. Somit kann die Lanzette auch nach Gebrauch der Lanzettenvorrichtung, eine definierte Rückzugstrecke durchlaufend, wiederum sicher im Buchsenelement und ausreichend tief in diesem aufgenommen werden, so daß sichergestellt ist, daß die Spitze der Lanzette auch unbeabsichtigt nicht mehr berührt werden kann.

Wie Eingangs schon erwähnt, können die mit der Handhabe der Steckverbindung zusammenwirkenden Mittel zur Erzeugung eines Impulses, mit dem die Lanzette bei Auslösung der Lanzettenvorrichtung in die Haut eines Probanden eintrifft, konstruktiv vielfältiger Art sein. Ganz besonders vorteilhaft ist es aber, die Handhabe des Steckelements wenigstens teilweise als federelastischen Körper nach Art einer Membran auszubilden, die die Lanzette über das Steckelement nach Überwindung eines membranseitig einstellbaren Druckpunktes mit einem vorbestimmbaren Impuls in die Haut eintreten läßt. Dadurch wird auf konstruktiv einfache Weise die Auslösung des Punktierungsvorganges mit der erfindungsgemäßen Lanzettenvorrichtung völlig frei von den individuellen Eigenheiten der die Lanzettenvorrichtung benutzenden Personen bewirkt, d.h. frei von Kriterien, die diese Funktion beeinflussen könne, da die Bedienungsperson lediglich für die Überwindung des Druckpunktes der federelastischen Membran zu sorgen hat. Nach Überwindung des Druckpunktes wird ausschließlich durch die Federkonstante der Membran der Impuls bestimmt, mit dem die vom Steckelement getragene Lanzettenspitze vorschnellt und in die Haut eines Probanden in vorbestimmbare Tiefe eindringt.

Vorzugsweise ist dabei die Membran als im wesentlichen tellerförmiger Körper ausgebildet, der im Querschnitt konkav gewölbt ist, wobei die konkave Wölbung im ungebrauchten Zustand der Lanzettenvorrichtung axial vom Buchsenelement wegstehend ausgebildet ist.

Zwar ist es grundsätzlich möglich, die Membran auf beliebige geeignete Weise auszubilden und herzustellen, vorteilhaft, weil die Herstellungskosten der Lanzettenvorrichtung vermindernd, ist es jedoch, die Membran und das schaftartig ausgebildete Steckelement als integrales Formteil auszubilden, so daß prinzipiell die Lanzettenvorrichtung nur aus dem Steckelement mit Membran, dem Buchsenelement, in der das Steckelement aufgenommen wird, und dem Hülsenelement sowie der metallischen Lanzette besteht.

Wie aufgabengemäß gefordert, soll die erfindungsgemäße Lanzettenvorrichtung möglichst kostengünstig herstellbar sein, um ihre möglichst breite Anwendung zu ermöglichen und auch Probanden erreichen, denen bisher die Möglichkeit, die gattungsgemäßen Lanzettenvorrichtungen zu benutzen, verschlossen geblieben sind. Unter diesem Gesichtspunkt ist es außerordentlich vorteilhaft, die Handhabe des Buchsenelementes und die Handhabe des Steckelementes mit einer jeweils tellerförmigen Struktur zu versehen und im Zuge der Ausbildung der Verbindung beider Handhaben über jeweils an diesen ausgebildete Eingriffsmittel miteinander zu verbinden. Es sind also keine konstruktiv aufwendigen Verbindungsmittel nötig, vielmehr sind die beiden Handhabenteile, die erst gemeinsam die eigentliche Handhabe der Lanzettenvorrichtung bilden, auf vorbeschriebene einfache Weise verbindbar, ohne das zusätzliche Verbindungsmittel, Rastmittel oder Klebemittel vorgesehen werden müssen. Die Membran, die durch die Bedienungsperson, beispielsweise durch Druck auf die Membran mittels des Daumens der Bedienungsperson in Richtung der Lanzettenspitze gedrückt und bis zum Erreichen des voreingestellten Druckpunktes elastisch verformt wird und bei dieser Verformung eine geringfügige Durchmesservergrößerung bzw. allgemein eine Außendimensionsvergrößerung der Membran zur Folge hat, und diese nicht zu behindern, was einen negativen Einfluß auf den zu erreichen gewünschten Impuls vorbestimmter Größe hat, ist das Eingriffsmittel an der Handhabe des Buchsenelementes in Form einer im wesentlichen umlaufenden Nut ausgebildet und das Eingriffsmittel an der Handhabe des Steckelementes ist in Form einer im wesentlichen umlaufenden Feder ausgebildet, wobei zur Verbindung beider Handhaben die Feder in die Nut eingreift, wobei aber vorzugsweise die Verbindung der Handhaben, d.h. bei Eingriff der Feder in die Nut, die Verbindung dennoch ein geringes radiales Spiel zuläßt, so daß die besagte Durchmesser- bzw. Dimensionsvergrößerung der Membran bis zum Erreichen des Druckpunktes ohne Nachteile für den Impuls vonstattengehen kann.

Schließlich ist es vorteilhaft, das Steckelement, das Buchsenelement sowie das Hülsenelement aus Kunststoff, beispielsweise spritzfähigem Kunststoff in Form von Polypropylen, auszubilden.

Gegebenenfalls kann es vorteilhaft sein, das Hülsenelement aus einem anderen spritzfähigen Kunststoffwerkstoff auszubilden als dem des Steckelementes und dem des Buchsenelementes, um ggf. der spezifischen, gewünschten elastischen Verformbarkeit des Hülsenelementes in radialer Richtung geeigneterer Rechnung tragen zu können.

Die Erfindung wird nun unter Bezugnahme auf die nachfolgenden schematischen Zeichnungen anhand eines Ausführungsbeispieles im einzelnen eingehend beschrieben. Darin zeigen:
- Fig 1.: im wesentlichen im Schnitt eine Lanzettenvorrichtung, die sich in unausgelöstem Ruhezustand befindet,
- Fig 2.: einen Ausschnitt der Darstellung von Fig. 1 in der Seitenansicht
- Fig 3.: im wesentlichen im Schnitt die Lanzettenvorrichtung in ausgelöstem Zustand, bei dem die Lanzettenspitze in die Haut eingedrungen ist,
- Fig 4.: Aussicht auf die Lanzettenvorrichtung gemäß Fig. 3 von unten,
- Fig 5.: einen Ausschnitt der Darstellung von Fig. 3 in der Seitenansicht und
- Fig 6.: im wesentlichen im Schnitt die Lanzettenvorrichtung nach einmaliger Auslösung in verriegeltem Zustand mit in das Buchsenelement hin eingezogener Lanzettenspitze.

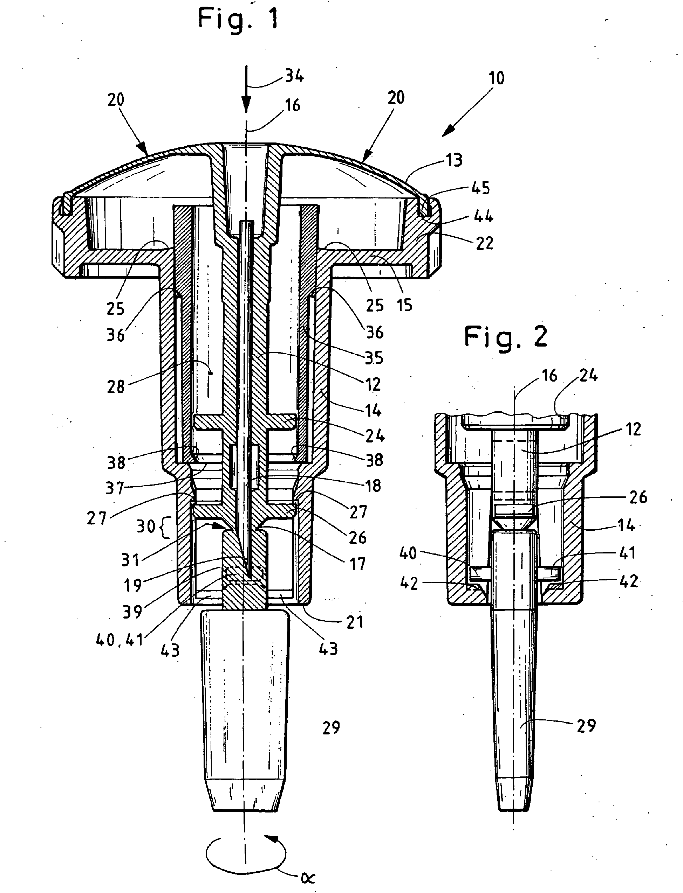

Es wird zunächst auf Fig. 1 Bezug genommen, anhand derer zunächst der grundsätzliche Aufbau der Lanzettenvorrichtung 10 beschrieben wird.

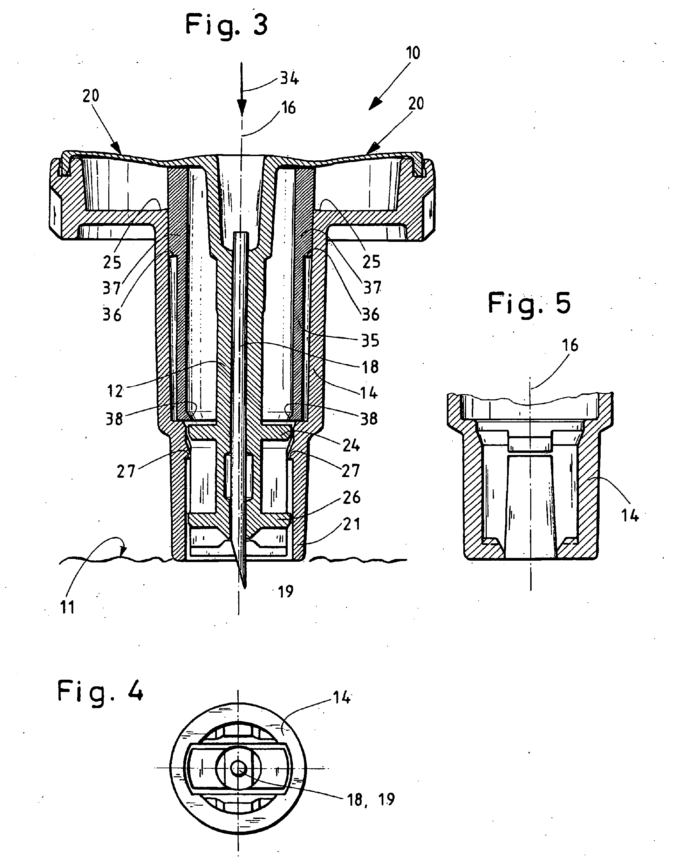

Die Lanzettenvorrichtung 10 besteht im wesentlichen aus einem Buchsenelement 14, das einen im wesentlichen kreisförmigen Querschnitt aufweisen kann, aber im Querschnitt auch beispielsweise oval oder elliptisch ausgeformt sein kann. Das Buchsenelement 14 weist einen Innenraum 28 auf. Im Innenraum 28 des Buchsenelementes 14 ist ein bei kreisförmigen Innenraumquerschnitt des Buchsenelementes 14 ebenfalls im wesentlichen im Hinblick auf seinen Querschnitt kreisförmig aufgebauten Hülsenelement 35 angeordnet, wobei das Hülsenelement 35 im Innenraum 28 längs einer die Lanzettenvorrichtung 10 durchlaufenden zentralen Achse 16 in Richtung des Pfeiles 34 und in entgegengesetzter Richtung hin und her verschiebbar ist. Zentral zur Achse 16 ist ein Steckelement 12 im Buchsenelement 14 aufgenommen, das ebenfalls in Richtung des Pfeiles 34 und in entgegengesetzter Richtung im Innenraum 28 bewegbar ist. Zentral zur Achse 16 ist im Steckelement 12 eine Lanzette 18 aufgenommen, die ein spitzes Ende 19 aufweist, im folgenden Lanzettenspitze 19 genannt, mit der die Lanzette 18 in die Haut 11 eines Säugetieres, insbesondere der eines Menschen, in bestimmungsgemäßer Tiefe bei dem bestimmungsgemäßen Gebrauch der Lanzettenvorrichtung 10 eindringt, vergleiche in diesem Falle Fig. 3.

Das Buchsenelement 14 ist mit einer Handhabe 15 versehen, die im wesentlichen tellerförmig ausgebildet sein kann. Die Handhabe 15 weist einen im wesentlichen umlaufenden Rand 22 auf, an dessen vertikal oberen Ende (in bezug auf die Darstellung in den Figuren) ein Eingriffsmittel in Form einer Nut 44, die ebenfalls die Handhabe 15 im wesentlichen umläuft, vorgesehen ist. Die Handhabe 15 ist im wesentlichen als vom Buchsenelement 14 radial wegstehendes, flanschähnliches Element ausgebildet, an dessen freiem Ende besagter Rand 22 ausgebildet ist.

Im Innenraum 28 oberhalb des an das Hülsenelement 35 angrenzenden Bereichs der Handhabe 15 bzw. des Buchsenelementes 14 ist ein Buchsenansatz 25 vorgesehen, der bei kreisförmigem Querschnitt des Buchsenelementes 14 um den dort gebildeten Rand des Buchsenelementes 14 umlaufend ausgebildet sein kann.

Das Buchsenelement 14 ist zu seinem freien Ende 21 hin offen ausgebildet und weist (in bezug auf die Darstellung der Figuren) in seinem unteren Bereich einen weiteren Buchsenansatz 27 auf, der bei im Querschnitt kreisförmig ausgebildetem Buchsenelement 14 ebenfalls im Innenraum 28 kreisförmig umlaufend ausgebildet sein kann. Die Funktion des ersten Buchsenansatzes 25 und des zweiten Buchsenansatzes 27 wird im Zusammenhang der Schilderung des Zusammenwirkens mit dem einzelnen Elementen des Steckelementes 12 weiter unten beschrieben.

Das Steckelement 12 weist eine als Membran 20 ausgebildete Handhabe 13 auf und einen im wesentlichen zylindrischen, schaftartigen Körper, der achszentral 16 im Innenraum 28 des Buchsenelementes aufgenommen wird. Die als Membran 20 ausgebildete Handhabe 13 des Steckelementes 12 weist einen im wesentlichen tellerförmigen Querschnitt auf, wobei im Randbereich um diesen umlaufend ein Eingriffsmittel 45 in Form einer Feder ausgebildet ist, wobei die Feder 45 der Handhabe 13 des Steckelements 12 in die Nut 44 der Handhabe 15 des Buchsenelementes 14 eingreift. Die Nut 44 und die Feder 45 sind derart bemessen, daß die Verbindung der Handhaben 13, 14, die Fig. 1 zeigt die aus den erwähnten Einzelelementen komplett zusammengebaute Lanzettenvorrichtung 10, im zusammengebauten Zustand ein geringes radiales Spiel aufweist bzw. zuläßt. Die Notwendigkeit des geringen axialen Spiels wird im Zusammenhang mit der Beschreibung der Funktion der Lanzettenvorrichtung 10 insbesondere unter Berücksichtigung der Darstellung von Fig. 3, im einzelnen noch weiter unten beschrieben.

Das Steckelement 12 weist einen ersten, im wesentlichen das Steckelement radial umlaufenden Vorsprung 24 auf und einen zweiten flügelartig radial von der Achse 16 wegstehendem zweiten Vorsprung 26, wobei die Vorsprünge 24, 26 im wesentlichen parallel axial voneinander beabstandet sind. Der erste Vorsprung 24 ist im Bereich des Hülsenelementes 35 im Innenraum 28 angeordnet, wohingegen der zweite Vorsprung 26 in bezug auf die Darstellung der Fig. im unteren, zum freien Ende 21 des Buchsenelementes hin gerichteten Teil des Buchsenelementes 14 angeordnet ist. Bei Bewegung des Steckelementes 12 entgegen der Richtung des Pfeiles 34 kommt der zweite Vorsprung 26 an dem Buchsenansatz 27 zur Anlage, d.h. die Bewegungsmöglichkeit des Steckelementes 12 entgegen der Richtung des Pfeiles 34 ist durch den Buchsenansatz 27 begrenzt.

Die Membran 20 und das schaftartig ausgebildete Steckelement 12 sind integral ausgebildet. Das Steckelement 12 ist an seinem freien Ende 17 mit einem Abdeckelement 29 versehen. Das Abdeckelement 29 steht, vergleiche die Fig. 1 und 2, im unbenutzten Zustand der Lanzettenvorrichtung 10 aus dem freien, offenen Ende 21 des Buchsenelementes 14 heraus. Das Abdeckelement 29 ist derart beschaffen, daß es von dem Benutzer der Lanzettenvorrichtung 10 beispielsweise zwischen Daumen und Zeigefinger ergriffen werden kann. Das Abdeckelement 29 umhüllt die Lanzettenspitze 19 steril, d.h. während des Herstellungsvorganges des Steckelementes 12 wird die Lanzette 18 durch den das Steckelement 12 und in seiner Verlängerung in Form des Abdeckelement 29 bildenden Werkstoff hermetisch umhüllt, so daß die Lanzettenspitze 19 nach Abschluß des Fertigungsvorganges steril umhüllt ist. Das Steckelement 12 und das Abdeckelement 29 können als einstückiges Formteil ausgebildet sein. Im Bereich des Anschlusses 30 des Abdeckelementes 29 ist eine Sollbruchstelle 31 vorgesehen. Die Sollbruchstelle 31 wird durch eine radiale Verjüngung des Anschlußbereiches 30 gebildet, vergleiche insbesondere Fig. 1.

Das Abdeckelement 29 weist zudem an seinem in unausgelöstem Zustand der Lanzettenvorrichtung 10 im Buchsenelement 14 positionierten Ende 39 zwei vom Abdeckelement 29 im wesentlichen radial abstehende Verriegelungsstege 40, 41 auf. Am Ende 21 des Buchsenelementes 14 sind in Form zweier radial in Richtung der Achse 16 vorstehende Widerlager 42, vergleiche insbesondere Fig. 2, vorgesehen. Bei unausgelöstem Zustand der Lanzettenvorrichtung 10 würden bei Betätigung der Handhabe 13, 15 in Richtung des Pfeiles 34 die Verriegelungsstege 40, 41 an das Widerlager 42 anstoßen, so daß ein Austreten der Lanzettenspitze 19 aus dem freien Ende 21 des Buchsenelementes 14 verhindert wird. D.h. mit anderen Worten, daß dann, wenn das Steckelement 12 mit dem Abdeckelement 29 verbunden im Buchsenelement 14 positioniert ist, eine Betätigung bzw. ein Auslösen der Lanzettenvorrichtung 10 nicht möglich ist, da die Verriegelungsstege 40, 41 an dem Widerlager 42 anliegen und somit eine axiale Bewegung des Steckelementes 12 in Richtung des Pfeiles 34 nicht möglich ist.

Bei Verdrehung des Abdeckelementes 29 um die Achse 16 um einen Winkel α, der beispielsweise 90° sein kann, siehe auch Fig. 1, erreichen die Verriegelungsstege 40, 41 einen widerlagerfreien Abschnitt 43, vergleiche Fig. 1 und 2, im Bereich des freien Endes 21 des Buchsenelementes 14. Im Zuge der. Drehung des Abdeckelementes 29 um den Winkel α erfolgt eine körperliche Trennung zwischen dem Steckelement 12 und dem Abdeckelement 29 infolge des gezielten Brechens der Verbindung an der Sollbruchstelle 31. Entgegen einer geringen, in Richtung des Pfeiles 34 aufzubringenden Kraft kann dann das Abdeckelement 29, da die Verriegelungsstege 40, 41 dem widerlagerfreien Abschnitt 43 gegenüberstehen, aus dem freien Ende 21 des Buchsenelementes herausgezogen werden. Dabei wird dann auch das spitze Ende 19 der Lanzette 18 freigelegt und die Lanzettenvorrichtung 10 ist funktions- bzw. betriebsbereit.

Zur Auslösung der Lanzettenvorrichtung 10 wird eine Kraft auf die Membran 20 in Richtung des Pfeiles 34 ausgeübt, indem beispielsweise der Daumen eines Benutzers auf die Membran 20 drückt, wobei die Handhabe 15 des Buchsenelementes 14 zwischen zwei Fingern ergriffen wird. Da die Membran 20 in Form eines federelastischen Körpers ausgebildet ist, kann über das mit der Membran 20 einstückig verbundene Steckelement 12 nach Überwindung eines membranseitig einstellbaren Druckpunktes die Lanzette 18, die nunmehr mit ihrer Lanzettenspitze 19 frei ist, mit einem vorbestimmbaren Impuls in die Haut 11, vergleiche Fig. 3, in vorbestimmter Tiefe eindringen. Die Bewegung der Membran 20 in Richtung des Pfeiles 34 ist begrenzt durch das in Anlagekommen an das Hülsenelement 35. Durch geeignete Wahl der Konstruktion der Membran 20, des Hülsenelementes 35 sowie des Buchsenelementes 14 kann die Eintrittstiefe der Membranspitze in die Haut 11 definiert vorgegeben werden.

Im Zuge der Bewegung des Steckelementes 12 in Richtung des Pfeiles 34 war der erste Vorsprung 24 des Steckelementes 12 in der Darstellung der Fig. über den bzw. die am unteren Ende angebrachten Absätze 38 des Hülsenelementes 35 geglitten und kommt dann in axialer Richtung unterhalb der Absätze 38 zu liegen, vergleiche Fig. 3, die diesen Verschiebungsendzustand des Steckelementes 12 zeigt.

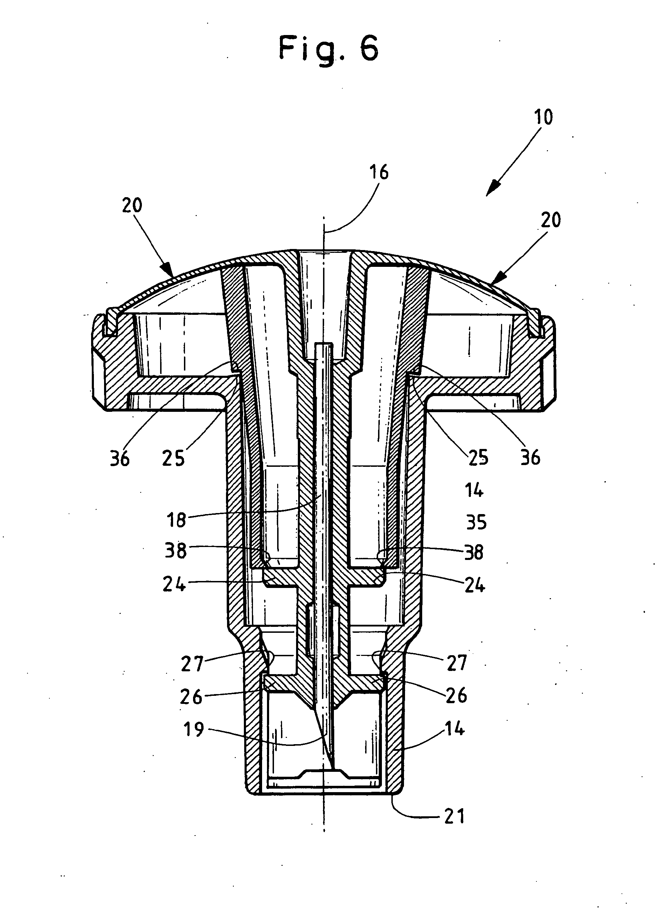

Wird nunmehr die Membran 20 nach Abschluß des Punktierungsvorganges losgelassen, erfolgt aufgrund der innewohnenden, gezielt eingestellten Vorspannung der Membran 20 eine axiale Bewegung der Membran 20 entgegen der Richtung des Pfeiles 34, da die Membran 20 dem unausgelösten Grundzustand, vergleiche Fig. 1 und 6, wieder einzunehmen sucht. Im Zuge dieser Rückbewegung des Steckelementes 12 entgegen der Richtung des Pfeiles 34 drückt der Vorsprung 24 des Steckelementes 12 das Hülsenelement 35 ebenfalls in entgegengesetzter Richtung des Pfeiles 34, d.h. in der Darstellung der Fig. nach oben, bis die Membran 20 ihre entspannte Grundstellung wieder erreicht hat, vergleiche Fig. 6.

Das Hülsenelement 35 weist an seinem dem ersten Absatz 38 im wesentlichen entgegengesetzten Ende 37 einen oben schon Vorsprung Absatz 36 auf, der nach Abschluß der axialen Verschiebung in entgegengesetzter Richtung des Pfeiles 34 hinter einem am Buchsenelement 14 innen ausgebildeten, ebenfalls oben schon erwähnten Buchsenansatz 35 greift, so daß die axiale Stellung des Hülsenelementes 35 eingenommen wird, wie sie in Fig. 6 dargestellt ist. Da der Buchsenansatz 25 nunmehr im Eingriff mit dem Absatz 38 des Hülsenelementes 35 steht und aufgrund der in radialer Richtung nach außen eingestellten Vorspannung des Hülsenelementes 35 keine Möglichkeit besteht, aus dieser Ineingriffstellung herauszukommen, ist der erfindungsgemäß angestrebte Verriegelungszustand nach einmaligem Gebrauch der Lanzettenvorrichtung 10 erreicht, da durch die Anlage des Hülsenelementes 35 innen an die Membran 20 keine nochmalige Bewegung der Membran 20 in Richtung des Pfeiles 34 möglich ist.

Das Steckelement 12, das Buchsenelement 14 und das Hülsenelement 35 können aus geeignetem, spritzfähigen Kunststoffwerkstoff, beispielsweise Polypropylen, hergestellt werden. Es sind aber grundsätzlich andere spritzfähige Kunststoffwerkstoffe zur Ausbildung dieser Elemente möglich und es ist auch möglich, die einzelnen voraufgeführten Elemente der Lanzettenvorrichtung 10 aus unterschiedlichen, geeigneten Kunststoffwerkstoffen herzustellen, um beispielsweise vorbestimmten elastischen Verformungskriterien besser Rechnung tragen zu können, beispielsweise denen des Hülsenelementes. Die Lanzette 18 selbst besteht regelmäßig aus einer geeigneten Stahllegierung und wird im Zuge des Herstellungsvorganges des Steckelementes, d.h. im Zuge des Spritzvorganges des Steckelementes 12, in dieses eingebettet.

### Bezugszeichenliste

- 10: Lanzettenvorrichtung
- 11: Haut
- 12: Steckelement
- 13: Handhabe
- 14: Buchsenelement
- 15: Handhabe
- 16: axial/ Buchsenachse/ Steckelementachse
- 17: freies Endes des Steck- elementes
- 18: Lanzette
- 19: spitzes Ende/ Lanzettenspitze
- 20: Membran
- 21: freies Ende des Membranelementes
- 22: Rand
- 23:
- 24: erster Vorsprung Steckelement
- 25: Buchsenansatz

- 26: zweiter Vorsprung
- 27: Buchsenansatz
- 28: Innenraum
- 29: Abdeckelement
- 30: Anschlußbereich
- 31: Sollbruchstelle
- 32:
- 33:
- 34: Pfeil/ Richtung/ Austrittsrichtung
- 35: Hülsenelement

- 36: Vorsprung
- 37: entgegengesetztes Ende

- 38: Absatz/ Buchsenabsatz
- 39: Ende Abdeckelement

- 40: Verriegelungssteg
- 41: Verriegelungssteg
- 42: Widerlager/ Buchsenelement
- 43: widerlagerfreier Abschnitt
- 44: Eingriffsmittel/ Nut
- 45: Eingriffsmittel/ Feder

## Patentansprüche

1. Lanzettenvorrichtung (10) zum Punktieren der Haut (11) von Säugetieren, insbesondere des Menschen, umfassend ein mit einer Handhabe (13) versehenes Steckelement (12) sowie ein mit einer Handhabe (15) versehenes Buchsenelement (14) zur inneren (28), axial (16) verschiebbaren Aufnahme des Steckelementes (12), wobei das Steckelement (12) an seinem von der Handhabe (13) weggerichteten freien Ende (17) eine mit einem spitzen Ende (19) versehene Lanzette (18) zum gezielten Eintritt in die Haut (11) aufweist, und wobei die Lanzette (18) über das Steckelement (12) durch eine in axialer (16) Richtung auf das freie Buchsenende (21) hin wirkende Kraft mit einem voreinstellbaren Impuls aus dem Buchsenende (21) heraus in die Haut (11) eintreten läßt, und daß Blockierungsmittel vorgesehen sind, die nach einmaligem Eintritt der Lanzette (18) in die Haut (11) die Möglichkeit eines nochmaligen Eintritts verhindern, **dadurch gekennzeichnet, daß** im Innenraum (28) des Buchsenelementes (14) ein axial in diesem verschiebbares Hülsenelement (35) angeordnet ist, das einen in wesentlichen auf die Achse (16) hin gerichteten Vorsprung (36) aufweist, über den hinweg ein von der Achse (16) weggerichteter erster Steckelementvorsprung (24) im Zuge des Eintritts der Lanzette (18) in die Haut (11) derart gleitend verschiebbar ist, daß er axial hinter den Vorsprung (36) greift und im Zuge des Wiedereintritts der Lanzette (18) in das Buchsenende (21) das Hülsenelement (35) axial (16) in Richtung der Handhabe (13) verschiebt.

2. Lanzettenvorrichtung nach Anspruch 1, **dadurch gekennzeichnet, daß** das Hülsenelement (35) an seinem dem ersten Vorsprung (36) im wesentlichen entgegengesetzten Ende (37) einen Absatz (38) aufweist, der nach Abschluß der axialen Verschiebung in Richtung der Handhabe (13) hinter einen am Buchsenelement (14) innen ausgebildeten Buchsenabsatz (38) greift.

3. Lanzettenvorrichtung nach Anspruch 2, **dadurch gekennzeichnet, daß** das Hülsenelement (35) wenigstens im Bereich seines Absatzes (38) radial von der Achse (16) weg vorgespannt ist.

4. Lanzettenvorrichtung nach einem oder mehreren der Ansprüche 1 bis 3, **dadurch gekennzeichnet, daß** das Steckelement (12) an seinem freien Ende (17) ein die daraus hervorstehende Lanzettenspitze (19) steril 1 umhüllendes Abdeckelement (29) aufweist.

5. Lanzettenvorrichtung nach Anspruch 4, **dadurch gekennzeichnet, daß** das Steckelement (12) und das Abdeckelement (29) als einstückiges Formteil ausgebildet ist.

6. Lanzettenvorrichtung nach einem oder beiden der Ansprüche 4 oder 5, **dadurch gekennzeichnet, daß** das Steckelement im Bereich des Anschlusses an das Abdeckelement (29) mit einer Sollbruchstelle (31) versehen ist.

7. Lanzettenvorrichtung nach Anspruch 6, **dadurch gekennzeichnet, daß** die Sollbruchstelle (31) durch eine radiale Verjüngung des Anschlußbereiches (30) gebildet wird.

8. Lanzettenvorrichtung nach einem oder mehreren der Ansprüche 4 bis 7, **dadurch gekennzeichnet, daß** das Abdeckelement (29) an seinem in unausgelöstem Zustand der Lanzettenvorrichtung (10) im Buchsenelement (14) positionierten Ende (39) im wesentlichen zwei vom Abdeckelement (29) im wesentlichen radial abstehende Verriegelungsstege (40, 41) aufweist, die im unausgelösten Zustand der Lanzettenvorrichtung (10) bei Betätigung der Handhabe (13, 15) in axialer (16) Richtung (34) an ein im Bereich des freien Endes (21) des Buchsenelementes (14) ausgebildetes Widerlager (42) stoßen.

9. Lanzettenvorrichtung nach Anspruch 8, **dadurch gekennzeichnet, daß** bei Verdrehung des Abdeckelementes (29) um eine Achse (16) um einen Winkel α die Verriegelungsstege (40, 41) einem widerlagerfreien Abschnitt (43) im Bereich des freien Endes (21) des Buchsenelementes (14) gegenüberstehen.

10. Lanzettenvorrichtung nach Anspruch 9, **dadurch gekennzeichnet, daß** bei Verdrehung des Abdeckelementes (29) um die Achse (16) um einen Winkel α die integrale Verbindung zwischen Abdeckelement (29) und Steckelement (12) gelöst ist und das Abdeckelement (29) aus dem Buchsenelement (14) entfernbar ist.

11. Lanzettenvorrichtung nach einem oder mehreren der Ansprüche 1 bis 10, **dadurch gekennzeichnet, daß** das Steckelement (12) einen von der Achse (16) weggerichteten zweiten, in Austrittsrichtung (34) der Lanzette (18) vor dem ersten Steckelementvorsprung (24) ausgebildeten Steckelementvorsprung (26) und axial zu diesem beabstandet aufweist, der den axialen (16) Verschiebungsweg der Lanzette (18) in das Buchsenelement (14) entgegen der Austrittsrichtung (34) durch in Anlage an einen Absatz (25) des Buchsenelementes (14) kommen begrenzt.

12. Lanzettenvorrichtung nach einem oder mehreren der Ansprüche 1 bis 11, **dadurch gekennzeichnet, daß** die Handhabe (13) des Steckelementes (12) wenigstens teilweise als federelastischer Körper nach Art einer Membran (20) ausgebildet ist, die die Lanzette (18) über das Steckelement (12) nach Überwindung eines membranseitig einstellbaren Druckpunktes mit einem vorbestimmbaren Impuls in die Haut (11) eintreten läßt.

13. Lanzettenvorrichtung nach Anspruch 12, **dadurch gekennzeichnet, daß** die Membran (20) als ein im wesentlichen tellerförmiger Körper ausgebildet ist, der im Querschnitt konkav gewölbt ist.

14. Lanzettenvorrichtung nach einem oder beider der Ansprüche 12 oder 13, **dadurch gekennzeichnet, daß** die Membran (20) und das schaftartig ausgebildete Steckelement (12) integral ausgebildet ist.

15. Lanzettenvorrichtung nach einem oder mehreren der Ansprüche 1 bis 14, **dadurch gekennzeichnet, daß** die Handhabe (15) des Buchsenelementes (14) und die Handhabe (13) des Steckelementes (12) jeweils eine im wesentlichen tellerförmige Struktur aufweisen und im Zuge der Verbindung der beiden Handhaben (13, 15) über jeweils an diesen ausgebildete Eingriffsmittel (44, 45) miteinander verbindbar sind.

16. Lanzettenvorrichtung nach Anspruch 15, **dadurch gekennzeichnet, daß** das Eingriffsmittel (44) an der Handhabe (15) des Buchsenelementes (14) in Form einer im wesentlichen umlaufenden Nut ausgebildet ist und das Eingriffsmittel (45) an der Handhabe (13) des Steckelementes (12) in Form einer im wesentlichen umlaufenden Feder ausgebildet ist, wobei zur Verbindung beider Handhaben (13, 15) die Feder in die Nut eingreift.

17. Lanzettenvorrichtung nach einem oder beiden der Ansprüche 15 oder 16, **dadurch gekennzeichnet, daß** die Verbindung der Handhaben (13, 15) ein radiales Spiel aufweist.

18. Lanzettenvorrichtung nach einer oder mehreren der Ansprüche 1 bis 17, **dadurch gekennzeichnet, daß** das Steckelement (12), das Buchsenelement (14) sowie das Hülsenelement (35) aus Kunststoffwerkstoff bestehen.

## Claims

1. Lancet device (10) for puncturing the skin (11) of mammals, in particular humans, comprising a plug-in element (12) provided with a handle (13) and a bush element (14) provided with a handle (15) in the interior (28) of which the plug-in element (12) can be accommodated so as to slide axially (16), and the plug-in element (12) has on its free end (17) directed away from the handle (13) a lancet (18) with a pointed end (19) for piercing the skin (11) at a specific point, which lancet (18) the skin (11) to be pierced via the plug-in element (12) due to a force acting in the axial direction (16) towards the free bush end (21) with a pre-definable impulse from the bush end (21), and blocking means are provided which prevent the possibility of the lancet (18) piercing the skin (11) again once it has pierced it once, **characterised in that** a sleeve element (35) is disposed in the interior (28) of the bush element (14) so as to be axially displaceable therein and has a projection (36) directed essentially towards the axis (16) beyond which a first plug-in element projection (24) directed away from the axis (16) can be displaced in a sliding movement as the lancet (18) punctures the skin (11) so that it locates axially behind the projection (36) and pushes the sleeve element (35) axially (16) in the direction of the handle (13) as the lancet (18) moves back into the bush end (21).

2. Lancet device as claimed in claim 1, **characterised in that**, at its end (37) disposed essentially opposite the first projection (36), the sleeve element (35) has a shoulder (38) which locates behind a bush shoulder (38) disposed on the inside of the bush element (14) when the axial movement in the direction of the handle (13) has terminated.

3. Lancet device as claimed in claim 2, **characterised in that** the sleeve element (35) is biased radially away from the axis (16), at least in the region of its shoulder (38) .

4. Lancet device as claimed in one or more of claims 1 to 3, **characterised in that** the plug-in element (12) has a cover element (29) at its free end (17) surrounding the lancet tip (19) projecting out from it in order to keep it sterile.

5. Lancet device as claimed in claim 4, **characterised in that** the plug-in element (12) and the cover element (29) comprise an integrally moulded part.

6. Lancet device as claimed in one or both of claims 4 or 5, **characterised in that** the plug-in element is provided with a breaking point (31) in the region of the connection to the cover element (29).

7. Lancet device as claimed in claim 6, **characterised in that** the breaking point (31) is provided in the form of a tapered region of the connecting region (30).

8. Lancet device as claimed in one or more of claims 4 to 7, **characterised in that**, at its end (39) positioned in the bush element (14) when the lancet device (10) is in the non-triggered state, the cover element (29) has essentially two locking webs (40, 41) projecting essentially radially out from the cover element (29) , which, when the lancet device (10) is in the non-triggered state, abut with a thrust bearing (42) disposed in the axial (16) direction (34) in the region of the free end (21) of the bush element (14) when the handle (13, 15) is operated.

9. Lancet device as claimed in claim 8, **characterised in that** when the cover element (29) is turned about an axis (16) by an angle `, the locking webs (40, 41) sit opposite a portion (43) in the region of the free end (21) of the bush element (14) without a thrust bearing.

10. Lancet device as claimed in claim 9, **characterised in that** when the cover element (29) is turned about the axis (16) by an angle`, the integral connection between the cover element (29) and plug-in element (12) is released and the cover element (29) can be removed from the bush element (14).

11. Lancet device as claimed in one or more of claims 1 to 10, **characterised in that** the plug-in element (12)has a second plug-in element projection (26) directed away from the axis (16) disposed upstream of the first plug-in element projection (24) in the extraction direction (34) of the lancet (18) and spaced axially apart from it, which restricts the axial (16) displacement path of the lancet (18) into the bush element (14) opposite the extraction direction (34) by abutting on a shoulder (25) of the bush element (14).

12. Lancet device as claimed in one or more of claims 1 to 11, **characterised in that** the handle (13) of the plug-in element (12) is provided in the form of an at least partially resiliently elastic body in the form of a membrane (20), which allows the lancet (18) to penetrate the skin (11) via the plug-in element (12) with a pre-definable impulse after overcoming a membrane-side adjustable pressure point.

13. Lancet device as claimed in claim 12, **characterised in that** the membrane (20) is provided in the form of an essentially dish-shaped body, which has a concavely cambered cross-section.

14. Lancet device as claimed in one or both of claims 12 or 13, **characterised in that** the membrane (20) and the shaft-type plug-in element (12) are of an integral design.

15. Lancet device as claimed in one or more of claims 1 to 14, **characterised in that** the handle (15) of the bush element (14) and the handle (13) of the plug-in element (12) are both of an essentially dish-shaped structure and can be connected to one another by means of respective locating means (44, 45) provided on them when the two handles (13, 15) are being connected to one another.

16. Lancet device as claimed in claim 15, **characterised in that** the locating means (44) on the handle (15) of the bush element (14) is provided in the form of an essentially circumferentially extending groove and the locating means (45) on the handle (13) of the plug-in element (12) is provided in the form of an essentially circumferentially extending spring, and the spring locates in the groove in order to connect the two handles (13, 15).

17. Lancet device as claimed in one or both of claims 15 or 16, **characterised in that** the handles (13, 15) are connected with a radial clearance.

18. Lancet device as claimed in one or more of claims 1 to 17, **characterised in that** the plug-in element (12), the bush element (14) and the sleeve element (35) are made from plastics.

## Revendications

1. Dispositif de lancette (10) pour perforer la peau (11) d'animaux mammifères, en particulier d'humains, comprenant un élément enfichable (12), muni d'une poignée (13), ainsi qu'un élément formant boîte (14), muni d'une poignée (15), pour la réception intérieure (28), avec possibilité de déplacement axial (16), de l'élément enfichable (12), l'élément enfichable (12) présentant, à son extrémité libre (17), opposée à la poignée (13), une lancette (18) munie d'une extrémité pointue (19), pour assurer la pénétration, à dessein, dans la peau (11), et la lancette (18), par l'intermédiaire de l'élément enfichable (12), pouvant, avec une impulsion pré-réglable, au moyen d'une force agissant en direction axiale (16) sur l'extrémité libre (21) de boîte, pénétrer dans la peau (11) en sortant de l'extrémité de boîte (21), et en ce que des moyens de blocage sont prévus, qui, après pénétration initiale de la lancette (18) dans la peau (11), empêchent la possibilité d'une nouvelle pénétration, **caractérisé en ce qu'**un élément formant douille (35) est disposé dans l'espace intérieur (28) de l'élément formant boîte (14), l'élément formant douille étant déplaçable axialement dans l'espace intérieur de l'élément formant boîte, l'élément formant douille présentant une saillie (36) orientée sensiblement dans l'axe (16), par l'intermédiaire de laquelle une première saillie d'élément enfichable (24), orientée à l'opposé de l'axe (16), est déplaçable avec glissement, au cours de la pénétration de la lancette (18) dans la peau (11), de manière qu'elle saisisse axialement à l'arrière la saillie (36) et, au cours de la réintroduction de la lancette (18) dans l'élément formant boîte (21), elle déplace l'élément formant douille (35) axialement (16) dans la direction de la poignée (13).

2. Dispositif de lancette selon la revendication 1, **caractérisé en ce que** l'élément formant douille (35) présente, sur son extrémité (37) sensiblement opposée à la première saillie (36), un décrochement (38) qui, après achèvement du déplacement axial en direction de la poignée (13), saisit à l'arrière un décrochement de boîte (38), réalisé intérieurement sur l'élément formant boîte (14).

3. Dispositif de lancette selon la revendication 2, **caractérisé en ce que** l'élément formant douille (35) est précontraint de manière à s'écarter radialement de l'axe (16), au moins dans la zone de son décrochement (38).

4. Dispositif de lancette selon l'une ou plusieurs des revendications précédentes, **caractérisé en ce que** l'élément enfichable (12) présente, au niveau de son extrémité libre (17), un élément formant couvercle (29), enveloppant de façon stérile la pointe de lancette (19) sortant de l'extrémité libre.

5. Dispositif de lancette selon la revendication 4, **caractérisé en ce que** l'élément enfichable (12) et l'élément formant couvercle (29) sont réalisés en tant que pièce moulée d'une seule pièce.

6. Dispositif de lancette selon l'une ou les deux des revendications 4 ou 5, **caractérisé en ce que** l'élément enfichable est muni d'un point destiné à la rupture (31), dans la zone du raccordement à l'élément formant couvercle (29).

7. Dispositif de lancette selon la revendication 6, **caractérisé en ce que** le point destiné à la rupture (31), est formé par une réduction radiale de la zone de raccordement (30).

8. Dispositif de lancette selon l'une ou plusieurs des revendications 4 à 7, **caractérisé en ce que** l'élément formant couvercle (29) présente, à son extrémité (39) positionnée dans l'élément formant boîte (14) lorsque le dispositif de lancette (10) est à l'état non déclenché, essentiellement deux nervures de verrouillage (40, 41), faisant saillie sensiblement radialement de l'élément formant couvercle (29), qui, lorsque le dispositif de lancette (10) est à l'état non déclenché, en cas d'actionnement de la poignée (13, 15), viennent buter, en direction (34) axiale (16), sur un contre-appui (42) réalisé dans la zone de l'extrémité libre (21) de l'élément formant boîte (14).

9. Dispositif de lancette selon la revendication 8, **caractérisé en ce que**, en cas de rotation de l'élément formant couvercle (29), d'un angle α autour d'un axe (16), les nervures de verrouillage (40, 41) sont placées en regard d'un tronçon (43) exempt de contre-appui, dans la zone de l'extrémité libre (21) de l'élément formant boîte (14).

10. Dispositif de lancette selon la revendication 9, **caractérisé en ce que**, en cas de rotation de l'élément formant couvercle (29), d'un angle α autour d'un axe (16), la liaison solidaire, entre l'élément formant couvercle (29) et l'élément enfichable (12) est rompue, et l'élément formant couvercle (29) est susceptible d'être retiré de l'élément formant boîte (14).

11. Dispositif de lancette selon l'une ou plusieurs des revendication 1 à 10, **caractérisé en ce que** l'élément enfichable (12) présente une deuxième saillie d'élément enfichable (26), orientée à l'opposé de l'axe (16), réalisée, dans la direction de sortie (34) de la lancette (18), devant la première saillie d'élément enfichable (24) et de façon axialement espacée par rapport à celle-ci, délimitant la course de déplacement en direction axiale (16) de la lancette (18) dans l'élément formant boîte (14), à l'encontre du sens de sortie (34), par la mise en appui sur un décrochement (25) de l'élément formant boîte (14).

12. Dispositif de lancette selon l'une ou plusieurs des revendication 1 à 11, **caractérisé en ce que** la poignée (13) de l'élément enfichable (12) est réalisé au moins partiellement sous forme de corps élastique, à la manière d'une membrane (20) qui laisse entrer dans la peau (11) la lancette (18), avec une impulsion pouvant être prédéterminée, par l'intermédiaire de l'élément enfichable (12), après avoir surmonté un point de pression réglable côté membrane.

13. Dispositif de lancette selon la revendication 12, **caractérisé en ce que** la membrane (20) est réalisée sous forme de corps sensiblement en forme de plateau, ayant une incurvation concave observée en coupe transversale.

14. Dispositif de lancette selon l'une ou les deux des revendications 12 ou 13, **caractérisé en ce que** la membrane (20) et l'élément à enfichable (12) réalisé à la manière d'une tige sont réalisés d'une seule pièce.

15. Dispositif de lancette selon l'une ou plusieurs des revendication 1 à 14, **caractérisé en ce que** la poignée (15) de l'élément formant boîte (14) et la poignée (13) de l'élément enfichable (12) présentent chacune une structure sensiblement en forme de plateau et, au cours de la liaison des deux poignées (13, 15), sont susceptibles d'être reliées ensemble par l'intermédiaire chaque fois de moyens de mise en prise (44, 45) réalisés sur celles-ci.

16. Dispositif de lancette selon la revendication 15, **caractérisé en ce que** le moyen de mise en prise (44), sur la poignée (15) de l'élément formant boîte (14) est réalisé sous la forme d'une gorge de pourtour, et le moyen de mise en prise (45), sur la poignée (135) de l'élément enfichable (12) est réalisé sous la forme d'une languette faisant pratiquement le pourtour, la languette s'engageant dans la gorge pour assurer la liaison des deux poignées (13, 15).

17. Dispositif de lancette selon l'une ou les deux des revendications 15 ou 16, **caractérisé en ce que** la liaison des poignées (13, 15) présente un jeu radial.

18. Dispositif de lancette selon l'une ou plusieurs des revendication 1 à 17, **caractérisé en ce que** l'élément enfichable (12), l'élément formant boîte (14), ainsi que l'élément formant douille (35) sont en matière synthétique.
